(19) Europäisches Patentamt
European Patent Office
Office européen des brevets

(11) **EP 4 349 955 A1**

(12) **EUROPEAN PATENT APPLICATION**
published in accordance with Art. 153(4) EPC

(43) Date of publication:
**10.04.2024 Bulletin 2024/15**

(21) Application number: **22811688.5**

(22) Date of filing: **27.05.2022**

(51) International Patent Classification (IPC):
*C12M 1/12* $^{(2006.01)}$     *C12M 3/00* $^{(2006.01)}$
*C12N 5/071* $^{(2010.01)}$     *C12N 5/077* $^{(2010.01)}$
*A23L 13/00* $^{(2016.01)}$

(52) Cooperative Patent Classification (CPC):
**A23L 13/00; C12M 1/12; C12M 3/00; C12N 5/06**

(86) International application number:
**PCT/KR2022/007575**

(87) International publication number:
**WO 2022/250497 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.05.2021 KR 20210068884**

(71) Applicant: **Sea With, Inc**
**Daegu 42988 (KR)**

(72) Inventors:
• **LEE, Hee Jae**
  **Dalseong-gun Daegu 43019 (KR)**
• **KEUM, Joon Ho**
  **Dalseong-gun Daegu 43019 (KR)**

• **CHOI, Kyeong Hun**
  **Busan 46539 (KR)**
• **SON, Ye Bin**
  **Dalseong-gun Daegu 43014 (KR)**
• **CHUN, Song Yi**
  **Dalseong-gun Daegu 43014 (KR)**
• **KIM, Su Ji**
  **Dalseong-gun Daegu 42995 (KR)**
• **CHO, Sung Chun**
  **Incheon 21986 (KR)**
• **KIM, Dasom**
  **Gwangmyeong-si Gyeonggi-do 14325 (KR)**
• **JUNG, Chisung**
  **Ansan-si Gyeonggi-do 15320 (KR)**

(74) Representative: **Maiwald GmbH**
**Elisenhof**
**Elisenstraße 3**
**80335 München (DE)**

(54) **SCAFFOLD FOR CELL CULTURE**

(57)     A cell culture scaffold of the present invention consists of a seaweed-derived component, is edible and has a pore size and stiffness that are suitable for cell culture, and a significantly excellent moisture-holding ability to enable long-term culture and efficient cell proliferation even when a high density of muscle stem cells are inoculated, and thus it may be effectively used for producing cultured meat.

**EP 4 349 955 A1**

[FIG. 1]

**Description**

[Technical Field]

The present invention relates to a scaffold for cell culture, which allows cells to be inoculated at high density, enables long-term cell culture due to high initial adhesion efficiency of cells, and has excellent cell proliferation efficiency.

[Background Art]

As interest in animal ethics and environmental pollution increases, the interest in cultured meat is growing. Cultured meat is a meat-like food prepared by culturing animal cells, and animal-like food prepared using plant protein is called a meat substitute.

In the production of cultured meat, cells, a medium, an incubator, and a cell culturing scaffold are important. Cells are the beginning of cultured meat, and a culture medium is a liquid that provides nutrients for cells to grow. Regarding the culture medium, the current challenge is to reduce dependence on bovine serum and lower the costs of producing a culture medium. An incubator is a space where cells grow by feeding on a culture medium, require a certain amount of stirring to effectively supply oxygen and remove waste products while requiring a detailed design to minimize damage to cells. A cell culture scaffold must have a structure that provides a space where cells constituting cultured meat are adhered and grown, promotes a process of differentiating cells into muscle, protects cells from shock in an incubator, and also allows easy migration of nutrients and waste. However, there is still a lack of research on a cell culture scaffold satisfying the above conditions.

[Disclosure]

[Technical Problem]

In the current situation where interest in cultured meat is increasing, the present inventors had tried to develop a cell culture scaffold effective in cultured meat production. As a result, a cell culture scaffold was prepared from edible seaweed through a process including sterilization, decellularization, layer separation, gel component recovery, rapid cooling, and freeze-drying. The cell culture scaffold has pores with a size of 40 to 200 $\mu$m, a stiffness of 8 to 17 kPa, a significantly excellent moisture content, and a hypha structure therein, so it was confirmed that it is suitable for muscle stem cell culture and muscle cell differentiation. In addition, when the cell culture scaffold is prepared in the form of beads, it was confirmed to be effective in culturing and proliferating muscle stem cells.

Therefore, the present invention is directed to providing a scaffold for cell culture and beads for cell culture, which consist of a seaweed-derived ingredient.

[Technical Solution]

As described above, to produce cultured meat, a cell culture scaffold is needed and the cell culture scaffold for producing cultured meat must have appropriate physical properties for cell culture to effectively induce differentiation of myocytes into muscle tissue. In addition, unlike a general cell culture scaffold, since muscle stem cells are initially seeded at high density, the cell culture scaffold according to the present invention preferably has excellent cell adhesion efficiency and consists of an edible ingredient.

As a result of studying a cell culture scaffold that can satisfy the above conditions, the present inventors developed a cell culture scaffold (hereinafter, referred to as an ACe-gel scaffold) prepared by removing the cortical portion of seaweed and freeze-drying the medullar layer, and cell culture beads (hereinafter, referred to as ACe-gel beads) prepared by gelating the medullary layer.

Specifically, the ACe-gel scaffold may be prepared by the following method:

    a) immersing seaweed in a 1 to 3% (w/v) sodium chloride (NaCl) solution for ozone treatment;
    b) cutting the ozone-treated seaweed to a predetermined size;
    c) immersing the cut seaweed in a solution containing an anionic detergent;
    d) separating the cortical portion by slowly shaking the solution in which the seaweed is immersed;
    e) washing the medullary layer separated from the cortical layer with PBS; and
    f) forming a scaffold for cell culture by lyophilizing the separated medullary layer.

In addition, the ACe-gel beads may be prepared by the following method:

a) immersing seaweed in a 1 to 3% (w/v) sodium chloride (NaCl) solution for ozone treatment;
b) cutting the ozone-treated seaweed to a predetermined size;
c) immersing the cut seaweed in a solution containing an anionic detergent;
d) separating the cortical layer by slowly shaking the solution in which the seaweed is immersed;
e) washing the medullary layer separated from the cortical layer with PBS; and
f) forming beads for cell proliferation by gelating the separated medullary layer.

According to one embodiment of the present invention, in the method of preparing the ACe-gel scaffold and the ACe-gel beads, the seaweed may be one or more selected from the group consisting of sea mustard, kelp, green laver, hijiki, and *Gloiopeltis tenax,* and preferably, sea mustard.

According to one embodiment of the present invention, in the preparation methods, a) the immersing of seaweed in a sodium chloride solution for ozone treatment is a process of appropriately expanding the tissue of a sea mustard sample, and the ozone treatment is a process of sterilizing microorganisms present on the surface of the sea mustard sample. Ozone may be treated for 30 seconds to 2 minutes, and preferably, 1 minute to 2 minutes. In the ozone treatment process, water pressure with a certain intensity or higher may be applied to allow uniform administration of ozone.

After the ozone treatment, sea mustard is cut to a certain size and immersed in a solution containing an anionic detergent. In the solution containing an anionic detergent, the concentration of the anionic detergent may be 0.05 to 1.5% (w/v), and preferably, 0.1 to 0.5% (w/v). The anionic detergent that can be used herein is sodium dodecyl sulfate (SDS), Triton X-100, or polyethylene glycol (PEG).

The d) the separating of the cortical layer by slowly shaking the solution in which the seaweed is immersed is a process of separating the cortical layer and the medullary layer of seaweed, and thereby, the cortical layer which is densely aligned in the sea mustard and the medullary layer having a loose cellulose structure are separated from each other.

In the present invention, the medullary layer may be mixed with an additive such as collagen, gelatin, poly-L-lysine, poly-D-lysine, L-monosodium glutamate, or microfibrous cellulose.

According to one embodiment of the present invention, the ACe-gel scaffold is prepared by lyophilizing the medullary layer of seaweed, whereas the ACe-gel beads is prepared by gelating the medullary layer of seaweed.

According to one embodiment of the present invention, the gelation includes immersing the washed medullary layer in a 10 to 500 mM calcium chloride ($CaCl_2$) aqueous solution; and washing the immersed medullary layer with PBS after predetermined time during the process of immersion in the calcium chloride aqueous solution. The calcium chloride aqueous solution has a concentration of preferably 50 to 200 mM.

According to one embodiment of the present invention, by immediately lyophilizing the medullary layer of seaweed, the ACe-gel scaffold has a porous structure consisting of a complex of alginic acid and hyphae, and therefore not only facilitates the migration, adhesion, and growth of cells in the scaffold, but also has a characteristic of well delivering nutrients and oxygen deep inside. In addition, when inoculating cells on a scaffold, gelation may be achieved by adding a gelation material to a medium, so the scaffold can be gelated according to cell culture conditions. Moreover, since the ACe-gel beads has a 3D structure, cells may effectively proliferate when inoculating muscle stem cells.

In the present invention, the ACe-gel scaffold and the ACe-gel beads may be further subjected to sterilization after preparation. The sterilization may use steam sterilization using hydrogen peroxide ($H_2O_2$), UV sterilization, or gamma sterilization, but the present invention is not limited thereto.

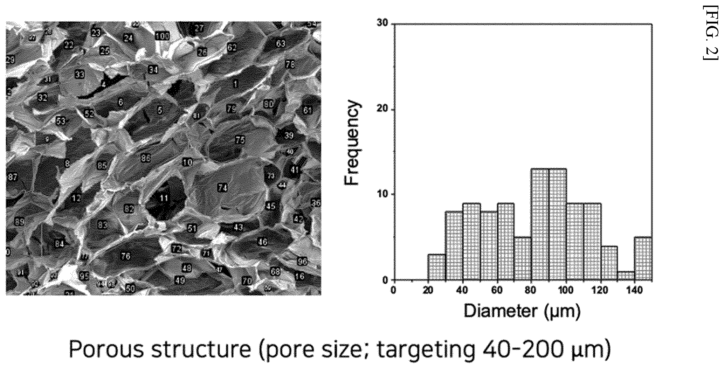

The ACe-gel scaffold according to one embodiment of the present invention is an effective scaffold for culturing muscle stem cells and producing cultured meat. Specifically, the ACe-gel scaffold consists of an algae-derived ingredient, so there is no need to separate the scaffold after producing cultured meat, and the ACe-gel scaffold has a pore of 40 to 200 $\mu$m, which is suitable for cell culture (FIG. 2).

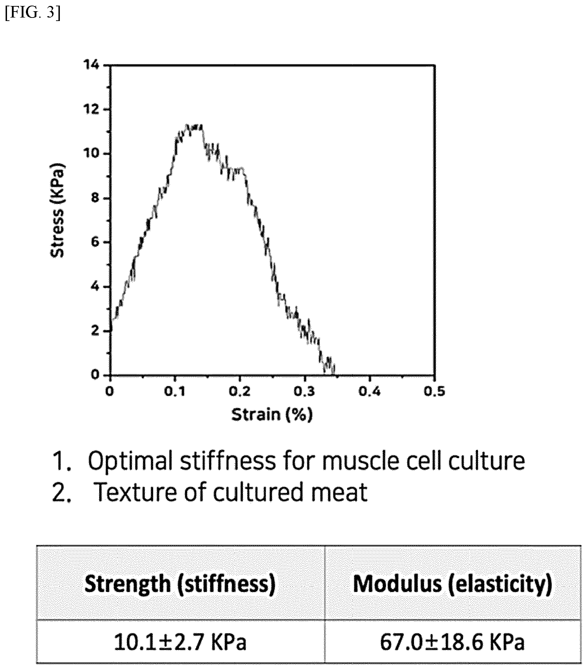

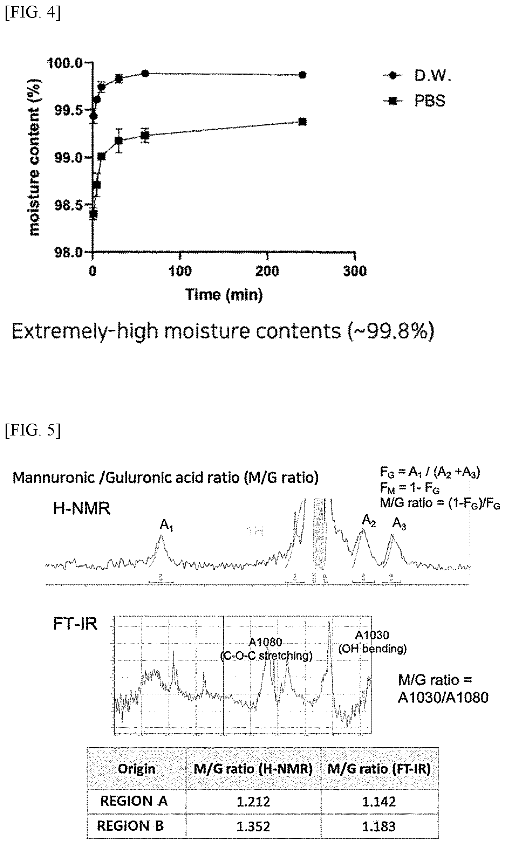

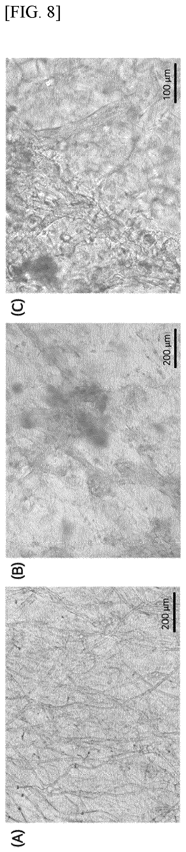

In addition, the ACe-gel scaffold has a hypha network structure, making it easy to attach and align muscle stem cells, and has excellent adhesion efficiency even when cells are initially inoculated at a high concentration, enabling long-term stable cell culture (FIGS. 8 to 10, and 12). The ACe-gel scaffold also has an excellent moisture-holding ability (FIG. 4), and stiffness suitable for myocyte culture (FIG. 3).

Meanwhile, since the ACe-gel scaffold and beads of the present invention are prepared by natural extraction of seaweed, as they do not undergo separation and purification like an existing alginate-based scaffold, the physical properties of the scaffold may vary according to the production area of seaweed. However, the content of alginate, which is the major component present in ACe-gel, and a mannuronic/guluronic acid ratio (M/G ratio) determining the stiffness of the scaffold may be measured to reduce the variation by production area (FIG. 5).

Accordingly, another aspect of the present invention provides a method of differentiating muscle stem cells into myocytes using an ACe-gel scaffold, and a method of producing cultured meat from muscle stem cells using ACe-gel scaffold and ACe-gel beads.

Specifically, the method of differentiating muscle stem cells into myocytes using the ACe-gel scaffold includes the following steps:

1) inoculating muscle stem cells into a scaffold for cell culture; and

2) culturing the muscle stem cells.

According to one embodiment of the present invention, the muscle stem cells may be inoculated on a scaffold for cell culture at a concentration of $0.5 \times 10^5$ to $5 \times 10^7$ cells/mL, and preferably, $1 \times 10^6$ to $3 \times 10^7$ cells/mL. The culturing step may proceed for 1 to 30 days.

Meanwhile, the method of producing cultured meat from muscle stem cells using the ACe-gel scaffold and ACe-gel beads includes the following steps:

1) inoculating muscle stem cells on the beads for cell culture and proliferating the cells;

2) collecting the proliferated muscle stem cells;

3) inoculating the collected muscle stem cells on the scaffold for cell culture; and

4) differentiating the muscle stem cells into myocytes.

To produce cultured meat, a very large amount of cells are needed, so generally, a sufficient amount of cells may not be obtained only using one cell culture scaffold. Accordingly, the present inventors invented a method of differentiating muscle stem cells into myocytes by sufficiently proliferating muscle stem cells by inoculating the muscle stem cells on ACe-gel beads and then inoculating the proliferated muscle stem cells on an ACe-gel scaffold.

In the present invention, the method of producing cultured meat may be performing by repeating 1) and 2) one or more times, preferably, two or more times, and more preferably, three times.

In the present invention, the muscle stem cells may be inoculated on the beads for cell culture at a concentration of $0.1 \times 10^5$ to $1 \times 10^6$ cells/mL, and preferably, $0.5 \times 10^5$ to $0. \times 10^6$ cells/mL.

In the present invention, 2) the collecting of the proliferated muscle stem cells may be performed using a conventional method known in the art. Specifically, an enzyme such as trypsin-EDTA, or Accutase may be used.

According to one embodiment of the present invention, the muscle stem cells may be inoculated on the scaffold for cell culture at $0.5 \times 10^5$ to $5 \times 10^7$ cells/mL, and preferably, $1 \times 10^6$ to $3 \times 10^7$ cells/mL. The culturing may be performed for 1 to 30 days.

In the present invention, 4) the differentiating of the muscle stem cells into myocytes may be performed by adding a differentiation-inducing material such as insulin to a medium, but the present invention is not limited thereto.

According to one embodiment of the present invention, as a result of culturing muscle stem cells by inoculation on each of the ACe-gel beads and the ACe-gel scaffold, it was confirmed that there are almost no dead cells and most muscle stem cells adhered and proliferated well. Accordingly, the ACe-gel beads and the ACe-gel scaffold may be effectively used for proliferating muscle stem cells, differentiating muscle stem cells into myocytes, and producing cultured meat.

[Advantageous Effects]

A cell culture scaffold of the present invention consists of a seaweed-derived component, is edible and has a pore size and stiffness that are suitable for cell culture, and a significantly excellent moisture-holding ability to enable long-term culture and efficient cell proliferation even when inoculating a high density of muscle stem cells, and thus it can be effectively used for producing cultured meat.

[Description of Drawings]

FIG. 1 schematically shows a process of preparing a cell culture scaffold (ACe-gel scaffold) and cell culture beads (ACe-gel beads) according to one embodiment of the present invention.

FIG. 2 is a result of confirming the pore size distribution of an ACe-gel scaffold.

FIG. 3 is a result of confirming the stiffness of an ACe-gel scaffold.

FIG. 4 is a result of confirming the moisture content of an ACe-gel scaffold.

FIG. 5 is a result of confirming the mannuronic/guluronic acid (M/G) ratios of seaweeds which have been grown in different production areas.

FIG. 6 is a result of confirming cell viability using a live/dead assay after culturing muscle stem cells on an ACe-gel scaffold.

FIG. 7 is a result of confirming cell viability using a live/dead assay after culturing muscle stem cells on an ACe-gel beads.

FIG. 8A is a result of confirming the hypha network structure of an ACe-gel scaffold, and FIGS. 8B and 8C are results of confirming that myocytes are attached and aligned according to the hypha network structure of Ace-gel scaffold.

FIG. 9 is a result of confirming cell proliferation efficiency 1, 4, 6, 14, and 21 days after seeding myocytes on an ACe-gel scaffold.

FIG. 10A is a result of confirming the alignment of myocytes after seeding the myocytes in an ACe-gel scaffold and performing3D culture.

FIG. 10B is a result of confirming the expression levels of genes expressed during myocyte differentiation.

FIG. 11 is an image of a cultured meat prototype prepared with an ACe-gel scaffold.

FIG. 12 is a result of confirming cell adhesion efficiency and long-term culture ability after seeding myocytes on an ACe-gel scaffold and Algimatrix (Thermo Fisher) at high-density.

[Modes of the Invention]

Hereinafter, one or more embodiments will be described in further detail with reference to examples. However, these examples are merely provided to illustrate one or more embodiments and thus the scope of the present invention is not limited to the following examples.

**Preparation Example: Preparation of cell culture scaffold (ACe-gel)**

Refrigerated salted sea mustard from Wando was washed under running water for 1 minute, and any part that was discolored or damaged was removed. After removing moisture from the sea mustard and measuring its weight, the sea mustard was immersed in a 1% NaCl solution (10 mL/g) for 5 minutes. The sea mustard was sterilized for 1 minute by ozone sterilization while immersed in the 1% NaCl solution. Here, water pressure above a certain intensity was applied to allow ozone to be uniformly administered. After removing the thick core of the sea mustard stem area, the sea mustard leaves were cut to a size of 3 *3 cm, and the cut sea mustard leaves were immersed in a 0.2% SDS solution (10 mL/g) and then shaken at 140 rpm for 10 minutes for decellularization. The resulting mixture was filtered through a mesh to remove the 0.2% SDS solution, the sea mustard was transferred to a tray, and distilled water (20 mL/g) was poured on the tray, shaken for 5 minutes at 10 rpm, and then rocked twice to remove remaining SDS by washing. After removing the distilled water through filtering with a mesh, the sea mustard was transferred to a beaker, distilled water (1 mL/g) was poured into a beaker, and then the resulting solution was shaken at 180 rpm for 30 minutes. The shaking speed was controlled to stimulate the entire sea mustard. After shaking, only a supernatant (medullar layer) was collected through centrifugation (5000 rpm, 15 min), and the lower mixture (cortical layer) was discarded. The collected supernatant may be mixed with an edible additive such as collagen, gelatin, poly-L-lysine, poly-D-lysine, L-sodium glutamate, or microfibrous cellulose. The collected supernatant was dispensed into a 24 well-plate or a square mold (28* 10*5 mm) at 500 uL/well or 1,600 uL. The supernatant was rapidly cooled by placing the plate or square mold in a deep freezer, and then freeze-dried using a lyophilizer. After freeze-drying, irregularities on the surface of the porous scaffold were removed with forceps. In addition, the collected supernatant was put in a 10 mL syringe with a 23G needle, and the supernatant was dropped into a beaker containing 50 mL of a 100 mM $CaCl_2$ crosslinking solution with the tip of a syringe needle spaced 20 cm apart from the crosslinking solution. Afterward, a bead-type scaffold was prepared by crosslinking for 30 minutes.

Hereinafter, the cell culture scaffold and the cell culture beads, which are prepared according to the method of this Preparation Example are referred to as "ACe-gel scaffold" and "ACe-gel beads," respectively.

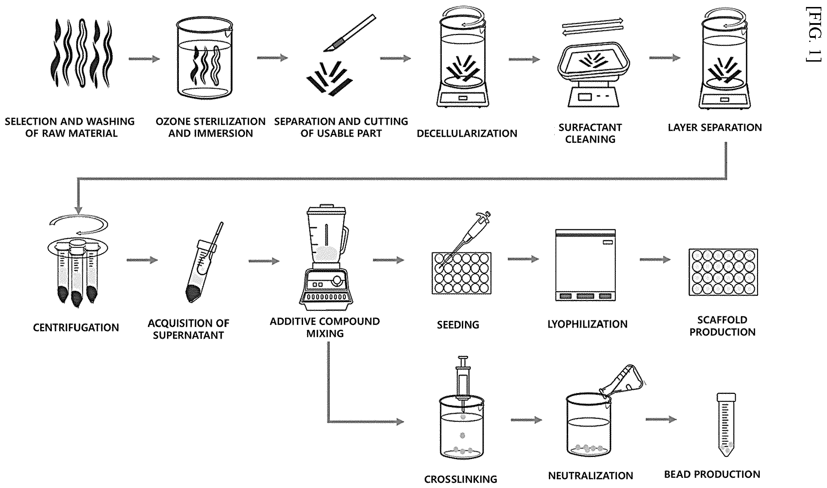

FIG. 1 illustrates the process of preparing the ACe-gel scaffold and the ACe-gel beads.

**Example 1: Confirmation of characteristics of ACe-gel**

**1-1. Pore size**

In order for cells to migrate into a scaffold to adhere and proliferate, it is known that the pore size of the scaffold is very important, and a pore distribution between 40 to 200 $\mu$m is suitable. Accordingly, the upper surface of the ACe-gel scaffold was photographed using a scanning electron microscope, and the sizes of 100 or more pores were analyzed using an ImageJ macro tool.

As a result of the analysis, it was confirmed that most of pores were 40 to 200 $\mu$m in size, which is suitable for cell culture, excluding errors considering the standard deviation (FIG. 2).

**1-2. Physical property (stiffness)**

The physical properties (e.g., stiffness) of the cell culture scaffold not only have a significant influence on the texture of cultured meat, but also have a significant influence on cells. For example, nerve cells and adipose cells require relatively soft microtissue elasticity, and muscle, cartilage, and bone cells require relatively high microtissue elasticity. Accordingly, the tensile properties of the ACe-gel scaffold were analyzed to determine whether the ACe-gel scaffold has an appropriate physical property for myocyte culture.

Specifically, an ACe-gel scaffold having a size of 9x24x4 mm (width x length x height) was tested under the following conditions: N=10+a; gauge length-10 mm; pretest speed-0.5 mm/s; test speed: 0.5 mm/s; date rate-50 points/s; trigger load-0.10 g; and load cell-10 kg.

As a result of the test, it was confirmed that the ACe-gel scaffold of the present invention has a stiffness of 8 to 17 kPa, suitable for the culture of myocytes (satellite cells) (FIG. 3).

## 1-3. Moisture content

For cell culture scaffolds, the ability to retain and hold moisture is important. Although there is no conventionally set standard, according to the reference (J. Mater. Chem. B, 2015, 3, 8401-8409), when the moisture content is 91.2% or more, it is judged to be a "high moisture content," and when the moisture content is 99.7% or more, it is judged to be an "extremely-high moisture content." The ACe-gel scaffold was immersed in PBS or distilled water (D.W.) for certain time, and the total increased weight was measured according to Equation 1 below.

[Equation 1]

$$Moisture\ content = \frac{W_s - W_d}{W_s}$$

$W_s$ = Weight of Swollen scaffold
$W_d$ = Weight of dried scaffold

As a result of the measurement, it was confirmed that the moisture content of the ACe-gel scaffold is significantly high regardless of the solvent (FIG. 4). Such a result was also confirmed from the result of testing by an accredited testing and analysis organization, and it can be seen that the ACe-gel scaffold had an excellent moisture-holding ability and had high safety, compared with GMP-grade products (Moisture item in Table 1).

[Table 1]

| Category | Dupont Novamatrix® | Ace-gel™ | Suitability |
|---|---|---|---|
| pH | 5.5-8.5 | 6.4 | O |
| Moisture | 85% | 98.9% | O |
| Protein | <0.3% | 0.2% | O |
| Lead (Pb) | <0.50 ug/g | 0.00 ug/g | O |
| Hydrargyrum (Hg) | <0.30 ug/g | 0.00 ug/g | O |
| Cadmium (Cd) | <0.20 ug/g | 0.00 ug/g | O |
| Arsenic (As) | <1.50 ug/g | 0.03 ug/g | O |
| Copper (Cu) | <0.90 ug/g | 0.00 mg/100g | O |
| Tin (Sn) | <1.80 ug/g | 0.00 ug/g | O |
| Manganese (Mn) | <9.00 ug/g | 0.00 mg/100g | O |

## 1-4. M/G ratio analysis

Since the ACe-gel scaffold is prepared by natural extraction of seaweed, it does not undergo separation and purification like the existing alginate-based scaffold (KR10-2151203). Therefore, to control the production area-dependent variation of seaweed, the content of alginate, which is the main component present in ACe-gel and a mannuronic/guluronic acid (M/G) ratio determining the stiffness of the scaffold were measured. Since only the G-block of M/G is involved in the ionic crosslinking of alginate, generally, it can be seen that the higher the G content, the stiffer the scaffold.

Alginate was extracted from seaweed in each production area, dried and , ground into powder, and transmittance was analyzed using the atr mode of Fourier-transform infrared spectroscopy (FT-IR). Afterward, the M/G ratio was calculated from an area ratio of the 1080 peak and the 1030 peak.

H-NMR was measured at approximately 90 °C after dissolving the alginate powder in $D_2O$ as follows, and a M/G ratio was calculated using 5.4 (a1), 4.55(A2) and 4.43(A3) peak ratios:

1) 0.5 g of alginate was moistened with 5 mL ethanol (96%) dissolved in 50 mL HCL.
2) The resulting solution was adjusting to pH 5.0 by NaOH and hydrolyzed at 100C reflux for 10 min.
3) The resulting product was cooled to room temperature, adjusted to pH 3.0 by HCl, and subjected to 100C reflux for 20 min.
4) The resulting product was cooled to room temperature, neutralized (pH 7) by NaOH, and precipitated in ethanol (96%).
5) The precipitate was dried overnight at 70 °C.
6) The resulting product was dissolved at 1% (w/v) in $D_2O$.
7) The resulting solution was filtered into an NMR tube through a Whatman 13-mm syringe filter (pore size 0.22 $\mu$m).
8) During acquisition, the sample temperature was 90 °C.

As a result of analysis, it was confirmed that there was a difference in M/G ratio according to the production area of seaweed, and by measuring this ratio, the characteristics of the seaweed by production area can be distinguished (FIG. 5).

**Example 2: Confirmation of cell culture ability of ACe-gel scaffold and beads**

2-1. Live/Dead assay

Bovine muscle stem cells (bMuSCs) extracted from Korean cattle were seeded on an ACe-gel scaffold or ACe-gel beads, and cultured in a Dulbecco's Modified Eagle Medium (DMEM) supplemented with 10% FBS and 1% P/S for 1 to 15 days. Afterward, the cells were stained using a live/dead assay kit, and observed with a 3D confocal microscope.

[Table 2]

| Cell type | Primary cell, Adhesion property |
|---|---|
| Cell origin | bMuSC [Bovine, Hanwoo (Korean cattle), muscle, Semi-membranous muscle (SM)] |
| Seeding concentration | $5\times10^6$/mL (scaffold), $1\times10^5$/mL (beads) |
| Culture medium | 10% FBS + Dulbecco's Modified Eagle Medium (DMEM) + 1% P/S |

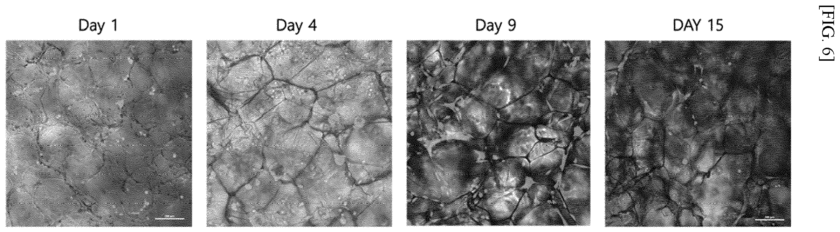

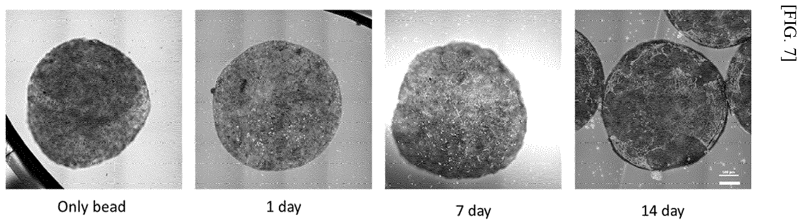

As a result of observation, it can be confirmed that there is mostly green fluorescence indicating live cells and almost no red fluorescence indicating dead cells, and muscle stem cells were well adhered along the hyphae of the ACe-gel scaffold and proliferated (FIG. 6). In addition, even when cultured on the ACe-gel beads, green fluorescence was shown in the majority of the cells, indicating that the muscle stem cells were well proliferated (FIG. 7).

The results demonstrated that the ACe-gel scaffold and ACe-gel beads are suitable for myocyte culture.

**2-2. Verification of muscle cell growth and differentiation**

To produce cultured meat, myocytes must differentiate into muscle tissue. Accordingly, myocytes (C2C12; mouse myoblast cell line) were seeded on the ACe-gel scaffold and 3D cultured, differentiation was confirmed using a microscope.

As a result of confirmation, it was confirmed that the ACe-gel scaffold of the present invention has a hypha network that can help with cell support and cell alignment (FIG. 8A), and myocytes are attached and aligned according to the hypha network structure (FIGS. 8B and 8C).

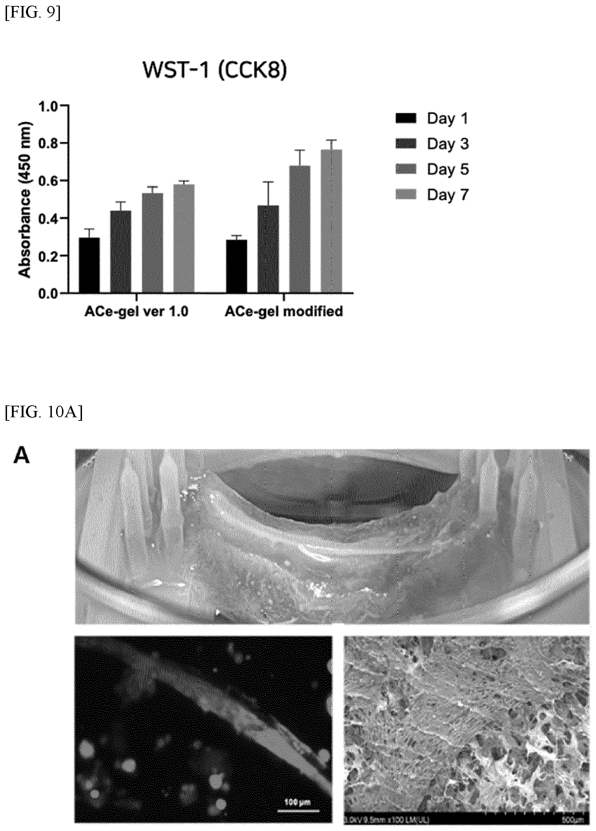

The 3D culture result demonstrated that cell proliferation ability of the ACe-gel scaffold is excellent (FIG. 9). In FIG. 9, ACe-gel ver 1.0 is a cell culture scaffold prepared according to Preparation Example without a separate additive, and ACe-gel modified is a food additive-added scaffold for further increasing cell proliferation efficiency.

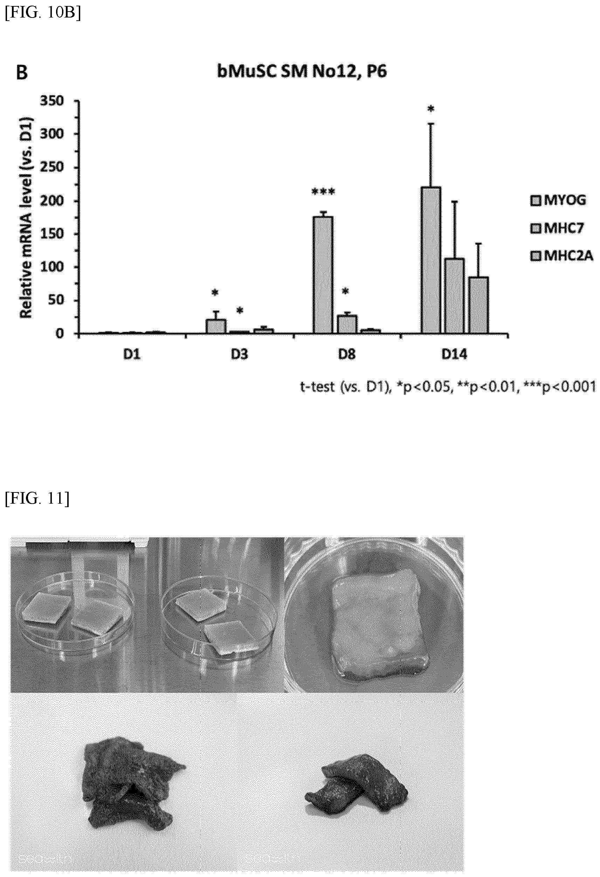

In addition, cell alignment required for the induction of muscle cell differentiation even in the 3D culture of bMuSCs was confirmed (FIG. 10A), and it was seen that the expression of genes during differentiation is also increased (FIG. 10B).

FIG. 11 shows an image of a cultured meat prototype prepared by culturing muscle stem cells on the ACe-gel scaffold.

**Comparative Example**

Myocytes (C2C12) were seeded at $2\times10^6$ cells/mL on an ACe-gel scaffold and Algimatrix (Thermo Fisher, U.S.A.), which is commercially available for research use, and 1, 4, 6, 14, or 21 days after seeding, the initial adhesion efficiency and long-term culture ability were confirmed by a CCK8 assay. The cell seeding concentration was much higher than the general cell seeding concentration on a 24-well plate because the lower the cell concentration, the longer it takes to proliferate and differentiate myocytes to produce cultured meat. Specific experimental methods are shown in Table 3 below.

[Table 3]

| C2C12 | |
|---|---|
| Number of passages | 21 |
| Cell counting | A total of $2.24\times10^8$ cells |
| Cell viability | 97.5% |
| Culture medium | High-DMEM+10% FBS+1% P/S |
| Seeding method | 24 well-round type: 200 $\mu$L medium+10 $\mu$L 20% CaCl$_2$ |
| Seeding density | $2\times10^6$ cells/mL (high density) |
| CCK8 assay | |
| CCK8 kit | Biomax Quantimax |
| Reaction time | 3 hours |
| Absorbance | Biomax (450 nm) |
| Loading volume | 20:1 (medium:reagent) 96 wells (100 $\mu$L) |
| Experimental group | ACe-gel scaffold Algimatrix |

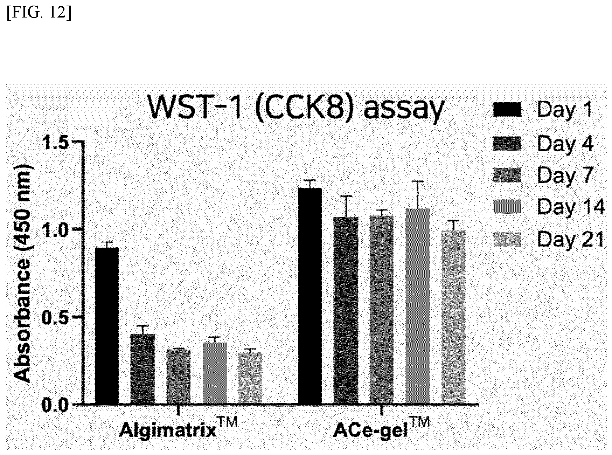

As a result of confirmation, compared with Algimatrix, when inoculated on the ACe-gel scaffold, it can be seen that the initial cell adhesion efficiency of myocytes is high, and even during long-term culture, the initial inoculated cell concentration remained almost constant, indicating that the cell maintenance ability was stabilized (FIG. 12).

Since it takes a long time to produce cultured meat by culturing myocytes, cells are seeded at a high density on a cell culture scaffold, and the cell culture scaffold must have an excellent ability to adhere and maintain a very high density of cells. The result of this comparative example demonstrates that the ACe-gel scaffold has a very excellent ability to adhere and maintain cells, so it is suitable for producing cultured meat.

**Claims**

1. A method of differentiating muscle stem cells into myocytes, comprising:

    1) inoculating muscle stem cells on a scaffold for cell culture; and
    2) culturing the muscle stem cells,

    wherein the scaffold for cell culture is prepared by

    a) immersing seaweed in a 0.1 to 3% (w/v) sodium chloride (NaCl) solution for ozone treatment;
    b) cutting the ozone-treated seaweed to a predetermined size;
    c) immersing the cut seaweed in a solution containing an anionic detergent;
    d) separating the cortical layer by slowly shaking the solution in which the seaweed is immersed;

e) washing the medullary layer separated from the cortical layer with phosphate buffered saline (PBS); and

f) preparing a scaffold for cell culture by lyophilizing the separated medullary layer.

2. The method of claim 1, wherein the muscle stem cells are inoculated at $0.5 \times 10^5$ to $5 \times 10^7$ cells/mL on the scaffold for cell culture.

3. The method of claim 1, wherein the muscle stem cells are cultured on the scaffold for cell culture for 1 to 30 days.

4. The method of claim 1, wherein the seaweed is one or more selected from the group consisting of sea mustard, kelp, green laver, hijiki, and *Gloiopeltis tenax.*

5. The method of claim 1, wherein the solution containing the anionic detergent has an anionic detergent concentration of 0.05 to 1.5% (w/v).

6. A method of preparing a scaffold for cell culture, comprising:

   a) immersing seaweed in a 1 to 3% (w/v) sodium chloride (NaCl) solution for ozone treatment;
   b) cutting the ozone-treated seaweed to a predetermined size;
   c) immersing the cut seaweed in a solution containing an anionic detergent;
   d) separating the cortical layer by slowly shaking the solution in which the seaweed is immersed;
   e) washing the medullary layer separated from the cortical layer with PBS; and
   f) preparing a scaffold for cell culture by lyophilizing the separated medullary layer.

7. A method of preparing beads for cell culture, comprising:

   a) immersing seaweed in a 1 to 3% (w/v) sodium chloride (NaCl) solution for ozone treatment;
   b) cutting the ozone-treated seaweed to a predetermined size;
   c) immersing the cut seaweed in a solution containing an anionic detergent;
   d) separating the cortical layer by slowly shaking the solution in which the seaweed is immersed;
   e) washing the medullary layer separated from the cortical layer with PBS; and
   f) preparing beads for cell culture for cell culture by gelating the separated medullary layer.

8. The method of claim 6 or 7, wherein the seaweed is one or more selected from the group consisting of sea mustard, kelp, green laver, hijiki, and *Gloiopeltis tenax.*

9. The method of claim 6 or 7, wherein the solution containing the anionic detergent has an anionic detergent concentration of 0.05 to 1.5% (w/v).

10. The method of claim 7, wherein the gelating of the separated medullary layer comprises

    immersing the washed medullary layer in a 10 to 500 mM calcium chloride ($CaCl_2$) aqueous solution; and
    washing the immersed medullary layer with PBS after a predetermined time during the process of immersion in the calcium chloride aqueous solution.

11. A scaffold for cell culture prepared by the method of claim 6.

12. Beads for cell culture prepared by the method of claim 7.

13. A method of producing cultured meat from muscle stem cells, comprising:

    1) inoculating muscle stem cells on the beads for cell culture prepared by the method of claim 7 and proliferating the cells;
    2) collecting the proliferated muscle stem cells;
    3) inoculating the collected muscle stem cells on the scaffold for cell culture prepared by the method of claim 6; and
    4) differentiating the muscle stem cells into myocytes.

14. The method of claim 13, wherein 1) and 2) are repeated one or more times.

**15.** The method of claim 13, wherein the muscle stem cells are inoculated on the beads for cell culture at $0.1 \times 10^5$ to $1 \times 10^6$ cells/mL.

**16.** The method of claim 13, wherein the muscle stem cells are inoculated on the scaffold for cell culture at $0.5 \times 10^5$ to $5 \times 10^7$ cells/mL.

[FIG. 1]

[FIG. 2]

Porous structure (pore size; targeting 40-200 μm)

[FIG. 3]

1. Optimal stiffness for muscle cell culture
2. Texture of cultured meat

| Strength (stiffness) | Modulus (elasticity) |
|---|---|
| 10.1±2.7 KPa | 67.0±18.6 KPa |

[FIG. 4]

Extremely-high moisture contents (~99.8%)

[FIG. 5]

Mannuronic /Guluronic acid ratio (M/G ratio)

$F_G = A_1 / (A_2 + A_3)$
$F_M = 1 - F_G$
M/G ratio = $(1-F_G)/F_G$

H-NMR

$A_1$   $A_2$   $A_3$

FT-IR

A1080
(C-O-C stretching)

A1030
(OH bending)

M/G ratio =
A1030/A1080

| Origin | M/G ratio (H-NMR) | M/G ratio (FT-IR) |
|---|---|---|
| REGION A | 1.212 | 1.142 |
| REGION B | 1.352 | 1.183 |

[FIG. 6]

[FIG. 7]

[FIG. 8]

[FIG. 9]

[FIG. 10A]

[FIG. 10B]

[FIG. 11]

[FIG. 12]

## INTERNATIONAL SEARCH REPORT

| | International application No. |
|---|---|
| | **PCT/KR2022/007575** |

**A. CLASSIFICATION OF SUBJECT MATTER**

**C12M 1/12**(2006.01)i; **C12M 3/00**(2006.01)i; **C12N 5/071**(2010.01)i; **C12N 5/077**(2010.01)i; **A23L 13/00**(2016.01)i

According to International Patent Classification (IPC) or to both national classification and IPC

**B. FIELDS SEARCHED**

Minimum documentation searched (classification system followed by classification symbols)

C12M 1/12(2006.01); A61L 27/36(2006.01); A61L 27/54(2006.01); C08J 3/075(2006.01); C08J 9/00(2006.01); C12N 5/00(2006.01); C12N 5/0775(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세포 배양(cell culture), 지지체(scaffold), 해조류(seaweed), 오존(ozone), 줄기세포(stem cells), 근육세포(muscle cell)

**C. DOCUMENTS CONSIDERED TO BE RELEVANT**

| Category* | Citation of document, with indication, where appropriate, of the relevant passages | Relevant to claim No. |
|---|---|---|
| X | KR 10-2151203 B1 (SEA WITH, INC) 02 September 2020 (2020-09-02)<br>See claims 1-3 and 5-11; paragraphs [0058]-[0064]; and figures 1 and 8. | 1-16 |
| A | KR 10-1721514 B1 (PUSAN NATIONAL UNIVERSITY INDUSTRY-UNIVERSITY COOPERATION FOUNDATION) 30 March 2017 (2017-03-30)<br>See claims 1-10. | 1-16 |
| A | US 2019-0060520 A1 (UNIVERSITY OF OTTAWA) 28 February 2019 (2019-02-28)<br>See claims 1-14. | 1-16 |
| A | KR 10-2018-0054439 A (GWANGJU INSTITUTE OF SCIENCE AND TECHNOLOGY et al.) 24 May 2018 (2018-05-24)<br>See entire document. | 1-16 |
| A | KR 10-2012-0086948 A (SEOUL NATIONAL UNIVERSITY R&DB FOUNDATION) 06 August 2012 (2012-08-06)<br>See entire document. | 1-16 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **31 August 2022** | **31 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.

**PCT/KR2022/007575**

| Patent document cited in search report | | | Publication date (day/month/year) | Patent family member(s) | | | Publication date (day/month/year) |
|---|---|---|---|---|---|---|---|
| KR | 10-2151203 | B1 | 02 September 2020 | WO | 2021-125529 | A1 | 24 June 2021 |
| KR | 10-1721514 | B1 | 30 March 2017 | None | | | |
| US | 2019-0060520 | A1 | 28 February 2019 | AU | 2017-218476 | A1 | 27 September 2018 |
| | | | | AU | 2017-218476 | B2 | 17 February 2022 |
| | | | | BR | 112018016050 | A2 | 26 December 2018 |
| | | | | CA | 3014256 | A1 | 17 August 2017 |
| | | | | CN | 109152863 | A | 04 January 2019 |
| | | | | EP | 3413939 | A1 | 19 December 2018 |
| | | | | IL | 260993 | A | 20 September 2018 |
| | | | | IL | 260993 | B | 31 August 2021 |
| | | | | IL | 284915 | A | 31 August 2021 |
| | | | | IL | 284915 | B | 01 February 2022 |
| | | | | JP | 2019-509723 | A | 11 April 2019 |
| | | | | JP | 2022-002536 | A | 11 January 2022 |
| | | | | JP | 6957486 | B2 | 02 November 2021 |
| | | | | MX | 2018009551 | A | 06 May 2019 |
| | | | | US | 11045582 | B2 | 29 June 2021 |
| | | | | US | 11167062 | B2 | 09 November 2021 |
| | | | | US | 2021-0001007 | A1 | 07 January 2021 |
| | | | | US | 2022-0016317 | A1 | 20 January 2022 |
| | | | | WO | 2017-136950 | A1 | 17 August 2017 |
| KR | 10-2018-0054439 | A | 24 May 2018 | WO | 2018-093069 | A1 | 24 May 2018 |
| KR | 10-2012-0086948 | A | 06 August 2012 | KR | 10-1240133 | B1 | 11 March 2013 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102151203

**Non-patent literature cited in the description**

- *J. Mater. Chem. B,* 2015, vol. 3, 8401-8409